(19) Europäisches Patentamt
European Patent Office
Office européen des brevets

(11) Veröffentlichungsnummer: **0 319 712 A1**

# (12) EUROPÄISCHE PATENTANMELDUNG

(21) Anmeldenummer: **88118360.2**

(22) Anmeldetag: **04.11.88**

(51) Int. Cl.4: **C07D 207/456 , C07D 405/12 , A01N 43/36**

(30) Priorität: **13.11.87 DE 3738591**

(43) Veröffentlichungstag der Anmeldung:
**14.06.89 Patentblatt 89/24**

(84) Benannte Vertragsstaaten:
**BE CH DE FR GB IT LI NL SE**

(71) Anmelder: **BAYER AG**

**D-5090 Leverkusen 1 Bayerwerk(DE)**

(72) Erfinder: **Wollweber, Detlef, Dr.**
**Paul-Ehrlich-Strasse 17**
**D-5600 Wuppertal 1(DE)**
Erfinder: **Krämer, Wolfgang, Dr.**
**Rosenkranz 25**
**D-5093 Burscheid 2(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

(54) **N-Substituierte Dichlormaleinimide.**

(57) Neue N-substituierte Dichlormaleinimide der allgemeinen Formel (I),

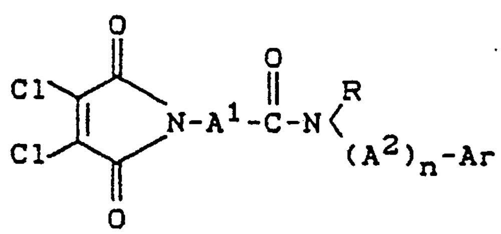

(I)

in welcher
R für Wasserstoff oder Alkyl steht,
Ar für gegebenenfalls substituiertes Aryl steht,
$A^1$ und $A^2$ unabhängig voneinander jeweils für gegebenenfalls substituiertes Alkylen stehen und
n für eine Zahl 0 oder 1 steht,
deren reine Isomere und Isomerengemische, ausgenommen die Verbindung 2-(Dichlormaleinimido)-aceta-

EP 0 319 712 A1

niilid, und die Verwendung zur Bekämpfung von Schädlingen.

Die Verbindungen der Formel (I) können nach Analogieverfahren hergestellt werden, so z.B. aus geeigneten Dichlormaleinimidocarbonsäurehalogeniden mit geeigneten Aminen.

## N-Substituierte Dichlormaleinimide

Die Erfindung betrifft neue N-substituierte Dichlormaleinimide, ein Verfahren zu ihrer Herstellung und ihre Verwendung als Schädlingsbekämpfungsmittel.

Es ist bekannt, daß bestimmte Dicarbonsäureimide wie beispielsweise die Verbindung N-Trichlormethansulfenylphthalsäureimid (Folpet) oder die Verbindung N-Trichlormethansulfenyl-tetrahydrophthalsäureimid (Captan) eine gute fungizide Wirksamkeit besitzen (vgl. z.B. K.H. Büchel "Pflanzenschutz und Schädlingsbekämpfung", S. 140, Thieme Verlag Stuttgart 1977).

Die Wirksamkeit dieser vorbekannten Verbindung ist jedoch insbesondere bei niedrigen Aufwandmengen und Konzentrationen nicht in allen Anwendungsgebieten völlig zufriedenstellend.

Außerdem ist die Verbindung 2-(Dichlormaleinimidoacetanilid bekannt (vgl. FR 2 005 669). Über eine Wirksamkeit dieser Verbindung gegenüber Schädlingen ist bisher nichts bekannt.

Es wurden neue N-substituierte Dichlormaleinimide der allgemeinen Formel (I),

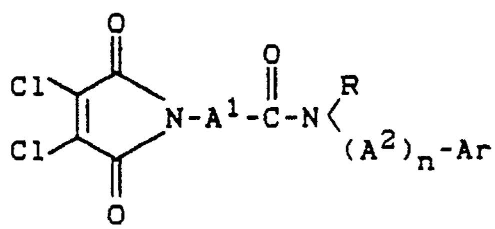

(I)

in welcher

R für Wasserstoff oder Alkyl steht,

Ar für gegebenenfalls substituiertes Aryl steht,

$A^1$ und $A^2$ unabhängig voneinander jeweils für gegebenenfalls substituiertes Alkylen stehen und

n für eine Zahl 0 oder 1 steht,

wobei die Verbindung 2-(Dichlormaleinimido)-acetanilid ausgenommen ist, gefunden.

Die Verbindungen der Formel (I) können in Abhängigkeit von der Art der Substituenten $A^1$ und/oder $A^2$ als optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen N-substituierten Dichlormaleinimide der allgemeinen Formel (I),

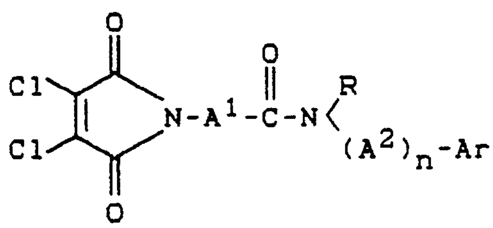

(I)

in welcher

R für Wasserstoff oder Alkyl steht,

Ar für gegebenenfalls substituiertes Aryl steht,

$A^1$ und $A^2$ unabhängig voneinander jeweils für gegebenenfalls substituiertes Alkylen stehen und

n für eine Zahl 0 oder 1 steht,

wobei die Verbindung 2-(Dichlormaleinimido)-acetanilid ausgenommen ist, erhält, wenn man Dichlormaleinimidocarbonsäurehalogenide der Formel (II),

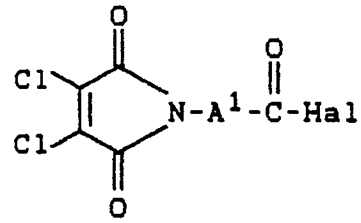

(II)

in welcher
A' die oben angegebene Bedeutung hat und
Hal für Halogen steht,
mit Aminen der Formel (III),

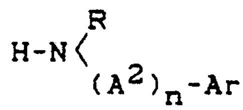

(III)

in welcher
R, Ar, $A^2$ und n die oben angegebene Bedeutung haben,
gegebenenfalls in Gegenwart eines Verdünnungsmittels und gegebenenfalls in Gegenwart eines Säurebindemittels umsetzt.

Schließlich wurde gefunden, daß die neuen N-substituierten Dichlormaleinimide der allgemeinen Formel (I) eine gute Wirkung gegen Schädlinge besitzen.

Überraschenderweise zeigen die erfindungsgemäßen N-substituierten Dichlormaleinimide der Formel (I) eine erheblich bessere fungizide Wirksamkeit als die aus dem Stand der Technik bekannten Dicarbonsäureimide, wie beispielsweise die Verbindung N-Trichlormethansulfenylphthalsäureimid (Folpet) oder die Verbindung N-Trichlormethansulfenyl-tetrahydrophthalsäureimid (Captan), welche chemisch und wirkungsmäßig naheliegende Verbindungen sind.

Die erfindungsgemäßen N-substituierten Dichlormaleinimide sind durch die Formel (I) allgemein definiert. Bevorzugt sind Verbindungen der Formel (I), bei welchen
R für Wasserstoff oder für geradkettiges oder verzweigtes Alkyl mit 1 bis 6 Kohlenstoffatomen steht,
Ar für gegebenenfalls einfach oder mehrfach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Dioxyhalogenalkylen oder Halogenalkylthio mit jeweils 1 bis 4 Kohlenstoffatomen und 1 bis 9 gleichen oder verschiedenen Halogenatomen, jeweils geradkettiges oder verzweigtes Alkenyl, Alkinyl, Alkenyloxy oder Alkinyloxy mit jeweils 3 bis 6 Kohlenstoffatomen, Cycloalkyl mit 3 bis 7 Kohlenstoffatomen, jeweils geradkettiges oder verzweigtes Alkoxycarbonylalkyl oder Alkoxyalkyl mit jeweils 1 bis 4 Kohlenstoffatomen in den einzelnen Alkylteilen oder jeweils gegebenenfalls einfach oder mehrfach, gleich oder verschieden durch Halogen und/oder geradkettiges oder verzweigtes Alkyl mit 1 bis 4 Kohlenstoffatomen substituiertes Phenyl, Benzyl, Phenoxy oder Benzyloxy,
A' und $A^2$ unabhängig voneinander jeweils für gegebenenfalls durch Phenyl substituiertes, geradkettiges oder verzweigtes Alkylen mit 1 bis 8 Kohlenstoffatomen stehen, wobei der Phenylsubstituent gegebenenfalls einfach oder mehrfach, gleich oder verschieden substituiert sein kann durch: Halogen, insbesondere Fluor, Chlor oder Brom, jeweils geradkettiges oder verzweigtes Alkyl, Alkoxy, Halogenalkyl oder Halogenalkoxy mit jeweils 1 bis 4 Kohlenstoffatomen und im Fall des Halogenalkyl bzw. Halogenalkoxy mit 1 bis 9 gleichen oder verschiedenen Halogenatomen und
n für eine Zahl 0 oder 1 steht,
wobei die Verbindung 2-(Dichlormaleinimido)-acetanilid ausgenommen ist.

Ganz besonders bevorzugt sind Verbindungen der Formel (I), bei welchen
R für Wasserstoff oder für geradkettiges oder verzweigtes Alkyl mit 1 bis 4 Kohlenstoffatomen steht,
Ar für gegebenenfalls ein- bis fünffach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Dioxydifluormethylen, Trifluormethylthio, Allyl, Propargyl, Cyclopropyl, Cyclohexyl, Phenyl, Benzyl, Phenoxy oder

Benzyloxy,
$A^1$ für gegebenenfalls einfach durch Phenyl substituiertes geradkettiges oder verzweigtes Alkylen mit 1 bis 6 Kohlenstoffatomen steht,
$A^2$ für unsubstituiertes geradkettiges oder verzweigtes Alkylen mit 1 bis 6 Kohlenstoffatomen steht und
n für eine Zahl 0 oder 1 steht,
wobei die Verbindung 2-(Dichlormaleinimido)-acetanilid ausgenommen ist.

Ganz besonders bevorzugt sind Verbindungen der Formel (I), bei welchen
R für Wasserstoff, Methyl oder Ethyl steht,
Ar für gegebenenfalls ein- bis dreifach, gleich oder verschieden substituiertes Phenyl steht, wobei als Substituenten infrage kommen: Fluor, Clor, Brom, Cyano, Nitro, Methyl, Ethyl, n- oder i-Propyl, n-, i-, s- oder t-Butyl, Methoxy, Methylthio, Trifluormethyl, Trifluormethoxy, Trifluormethylthio oder zweifach verknüpftes Dioxydifluormethylen,
$A^1$ für gegebenenfalls einfach durch Phenyl substituiertes geradkettiges oder verzweigtes Alkylen mit 1 bis 4 Kohlenstoffatomen steht,
$A^2$ für geradkettiges oder verzweigtes Alkylen mit 1 bis 4 Kohlenstoffatomen steht und
n für eine Zahl 0 oder 1 steht,
wobei die Verbindung 2-(Dichlormaleinimido)-acetanilid ausgenommen ist.

Im einzelnen seien außer den bei den Herstellungsbeispielen genannten Verbindungen die folgenden N-substituierten Dichlormaleinimide der allgemeinen Formel (I) genannt:

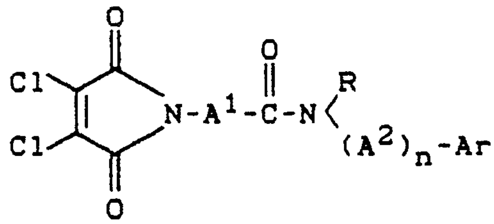

(I)

| $A^1$ | R | $(A^2)_n$ | Ar |
|---|---|---|---|
| $-CH_2-$ | H | - | 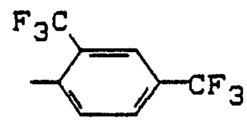 |
| $-CH_2$ | H | - | 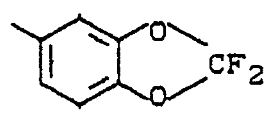 |
| $-CH_2-$ | H | $-CH_2-$ | 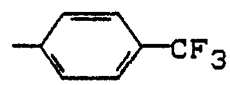 |
| $-CH_2-$ | H | $-(CH_2)_4-$ | 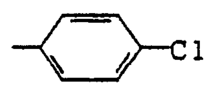 |
| $-CH_2-$ | H | $-(CH_2)_6-$ | 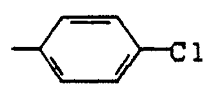 |
| $-CH_2-$ | H | $-CH_2-$ | 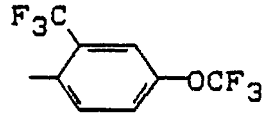 |

| $A^1$ | R | $(A^2)_n$ | Ar |
|---|---|---|---|
| $-CH_2-$ | H | $-CH_2-$ | 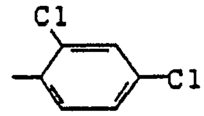 |
| $-CH_2-$ | H | $-(CH_2)_3-$ | 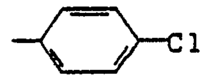 |
| $-CH_2$ | H | $-(CH_2)_5-$ | 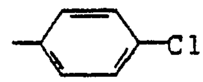 |
| $-(CH_2)_4-$ | H | - | 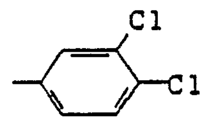 |
| $-(CH_2)_4-$ | H | - | 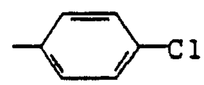 |
| $-(CH_2)_4-$ | H | - | 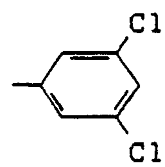 |
| $-CH_2-$ | H | - | 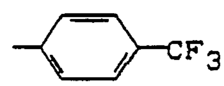 |
| $-CH_2-$ | H | - | 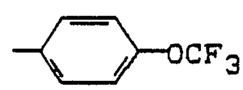 |

| $A^1$ | R | $(A^2)_n$ | Ar |
|---|---|---|---|
| $-CH_2-$ | H | - | 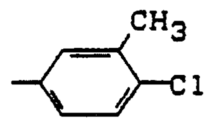 |
| $-CH_2-$ | H | - | 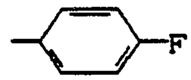 |
| $-CH_2-$ | H | - | 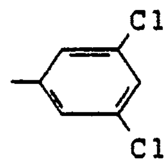 |
| $-CH_2-$ | H | - | 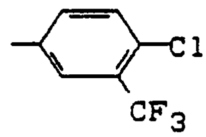 |
| $-CH_2-$ | H | - | 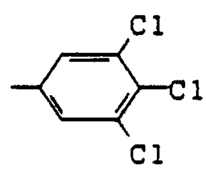 |
| $-CH_2-$ | H | - | 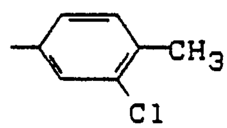 |
| $-CH_2-$ | H | - | 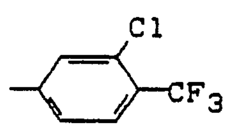 |

| $A^1$ | R | $(A^2)_n$ | Ar |
|---|---|---|---|
| $-CH_2-$ | H | $-CH_2-$ | 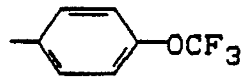 |
| $-CH_2-$ | H | $-CH_2-$ | 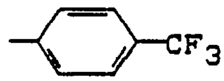 |
| $-CH_2-$ | H | $-CH_2-$ | 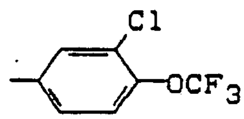 |
| $-CH_2-$ | H | $-(CH_2)_2-$ | 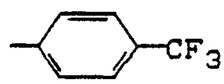 |
| $-CH_2-$ | H | $-(CH_2)_2-$ | 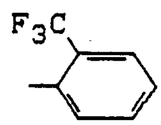 |
| $-CH_2-$ | H | $-(CH_2)_2-$ | 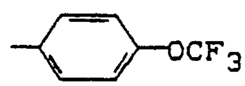 |
| $-(CH_2)_2-$ | H | - | 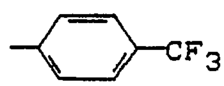 |
| $-(CH_2)_2-$ | H | - | 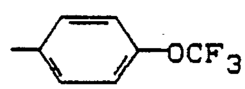 |

| $A^1$ | R | $(A^2)_n$ | Ar |
|---|---|---|---|
| $-(CH_2)_2-$ | H | - | 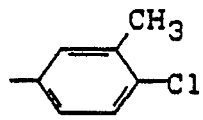 |
| $-(CH_2)_2-$ | H | - | 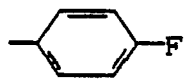 |
| $-(CH_2)_2-$ | H | - | 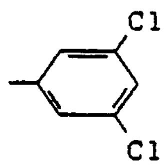 |
| $-(CH_2)_2-$ | H | - | 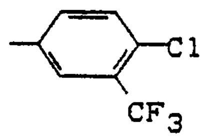 |
| $-(CH_2)_2-$ | H | - | 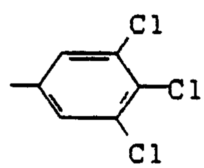 |
| $-(CH_2)_2-$ | H | - | 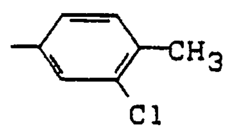 |
| $-(CH_2)_2-$ | H | - | 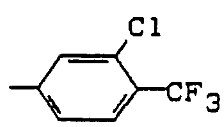 |

| $A^1$ | R | $(A^2)_n$ | Ar |
|---|---|---|---|
| $-(CH_2)_2-$ | H | $-CH_2-$ | 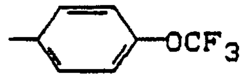 |
| $-(CH_2)_2-$ | H | $-CH_2-$ | 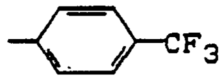 |
| $-(CH_2)_2-$ | H | $-CH_2-$ | 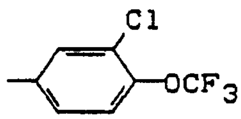 |
| $-(CH_2)_2-$ | H | $-(CH_2)_2-$ | 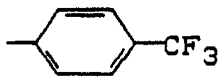 |
| $-(CH_2)_2-$ | H | $-(CH_2)_2-$ | 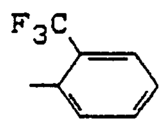 |
| $-(CH_2)_2-$ | H | $-(CH_2)_2-$ | 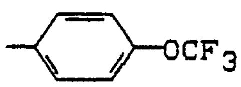 |
| $-(CH_2)_3-$ | H | - | 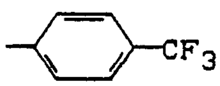 |
| $-(CH_2)_3-$ | H | - | 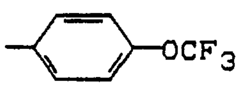 |

| $A^1$ | R | $(A^2)_n$ | Ar |
|---|---|---|---|
| $-(CH_2)_3-$ | H | - | 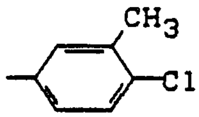 |
| $-(CH_2)_3-$ | H | - | 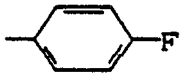 |
| $-(CH_2)_3-$ | H | - | 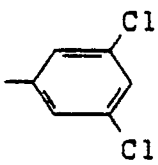 |
| $-(CH_2)_3-$ | H | - | 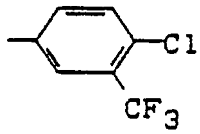 |
| $-(CH_2)_3-$ | H | - | 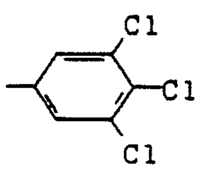 |
| $-(CH_2)_3-$ | H | - | 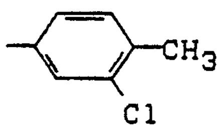 |
| $-(CH_2)_3-$ | H | - | 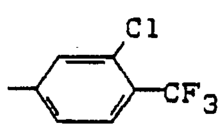 |

| $A^1$ | R | $(A^2)_n$ | Ar |
|---|---|---|---|
| $-(CH_2)_3-$ | H | $-CH_2-$ | 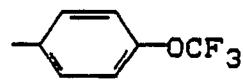 |
| $-(CH_2)_3-$ | H | $-CH_2-$ | 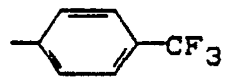 |
| $-(CH_2)_3-$ | H | $-CH_2-$ | 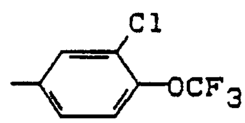 |
| $-(CH_2)_3-$ | H | $-(CH_2)_2-$ | 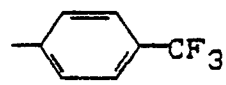 |
| $-(CH_2)_3-$ | H | $-(CH_2)_2-$ | 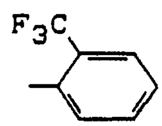 |
| $-(CH_2)_3-$ | H | $-(CH_2)_2-$ | 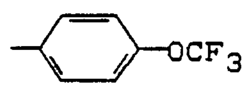 |
| $-CH(CH_3)-$ | H | - | 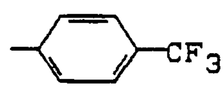 |
| $-CH(CH_3)-$ | H | - | 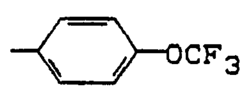 |

| $A^1$ | R | $(A^2)_n$ | Ar |
|---|---|---|---|
| 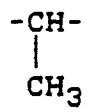 | H | - | 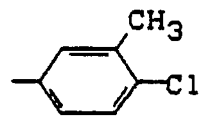 |
| 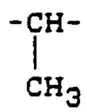 | H | - | 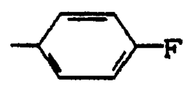 |
| 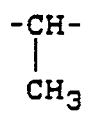 | H | - | 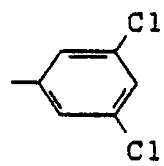 |
| 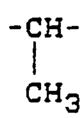 | H | - | 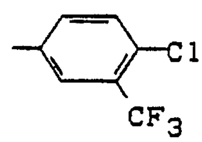 |
| 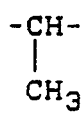 | H | - | 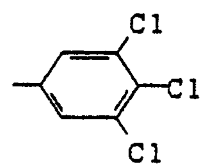 |
| 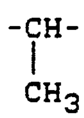 | H | - | 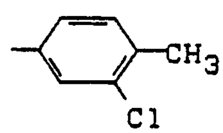 |
| 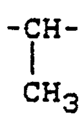 | H | - | 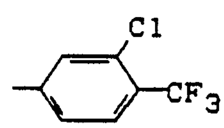 |

| $A^1$ | R | $(A^2)_n$ | Ar |
|---|---|---|---|
| 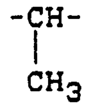 | H | $-CH_2-$ | 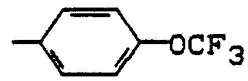 |
| 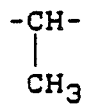 | H | $-CH_2-$ | 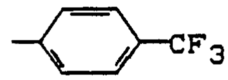 |
| 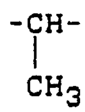 | H | $-CH_2-$ | 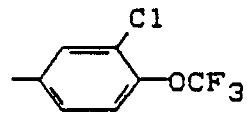 |
| 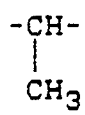 | H | $-(CH_2)_2-$ | 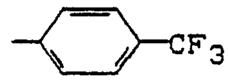 |
| 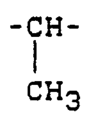 | H | $-(CH_2)_2-$ | 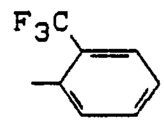 |
| 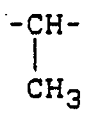 | H | $-(CH_2)_2-$ | 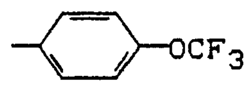 |
| 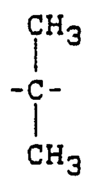 | H | - | 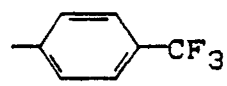 |

| $A^1$ | R | $(A^2)_n$ | Ar |
|---|---|---|---|
| 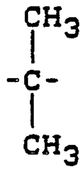 | H | - | 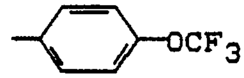 |
| 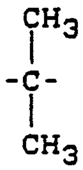 | H | - | 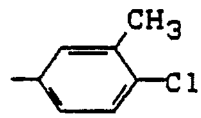 |
| 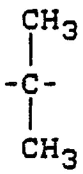 | H | - | 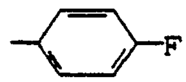 |
| 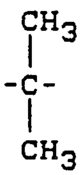 | H | - | 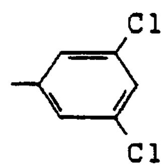 |
| 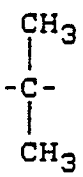 | H | - | 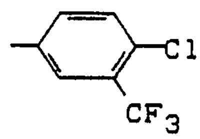 |
| 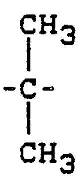 | H | - | 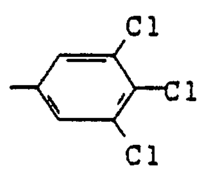 |

| $A^1$ | R | $(A^2)_n$ | Ar |
|---|---|---|---|
| 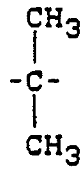 | H | - | 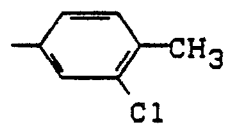 |
| 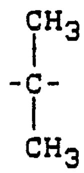 | H | - | 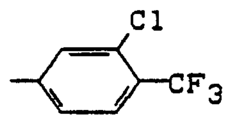 |
| 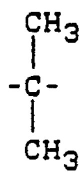 | H | $-CH_2-$ | 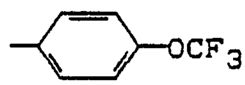 |
| 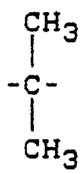 | H | $-CH_2-$ | 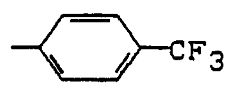 |
| 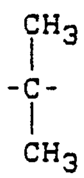 | H | $-CH_2-$ | 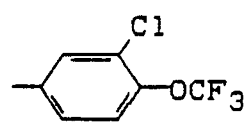 |
| 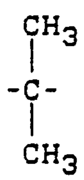 | H | $-(CH_2)_2-$ | 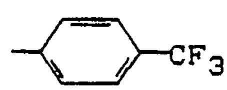 |

| $A^1$ | R | $(A^2)_n$ | Ar |
|---|---|---|---|
| 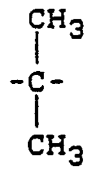 | H | $-(CH_2)_2-$ | 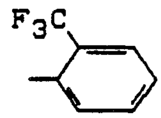 |
| 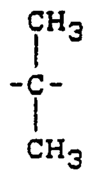 | H | $-(CH_2)_2-$ | 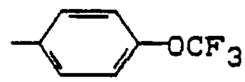 |
| 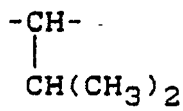 | H | - | 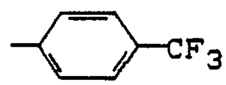 |
| 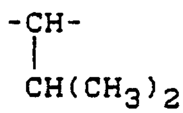 | H | - | 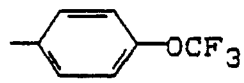 |
| 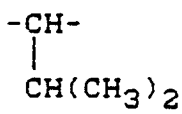 | H | - | 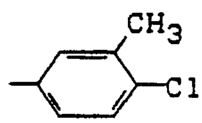 |
| 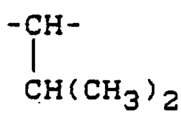 | H | - | 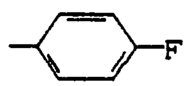 |
| 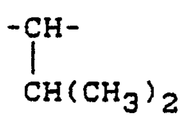 | H | - | 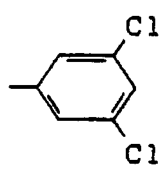 |

| $A^1$ | R | $(A^2)_n$ | Ar |
|---|---|---|---|
| $-CH-$<br>$CH(CH_3)_2$ | H | - | 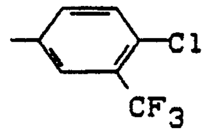 |
| $-CH-$<br>$CH(CH_3)_2$ | H | - | 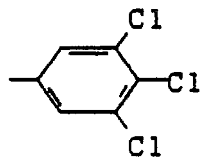 |
| $-CH-$<br>$CH(CH_3)_2$ | H | - | 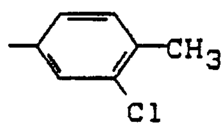 |
| $-CH-$<br>$CH(CH_3)_2$ | H | - | 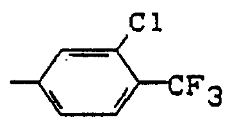 |
| $-CH-$<br>$CH(CH_3)_2$ | H | $-CH_2-$ | 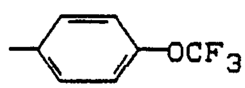 |
| $-CH-$<br>$CH(CH_3)_2$ | H | $-CH_2-$ | 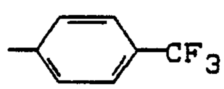 |
| $-CH-$<br>$CH(CH_3)_2$ | H | $-CH_2-$ | 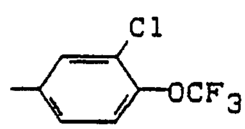 |

| $A^1$ | R | $(A^2)_n$ | Ar |
|---|---|---|---|
| $-CH-$<br>$CH(CH_3)_2$ | H | $-(CH_2)_2-$ | 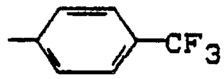 |
| $-CH-$<br>$CH(CH_3)_2$ | H | $-(CH_2)_2-$ | 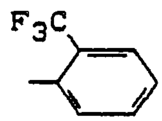 |
| $-CH-$<br>$CH(CH_3)_2$ | H | $-(CH_2)_2-$ | 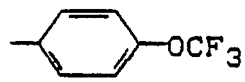 |
| 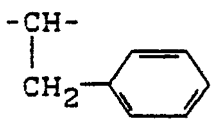 | H | - | 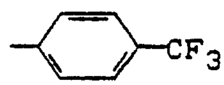 |
| 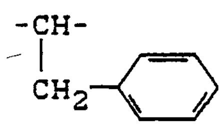 | H | - | 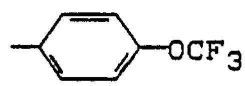 |
| 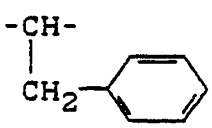 | H | - | 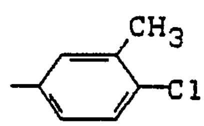 |

| $A^1$ | R | $(A^2)_n$ | Ar |
|---|---|---|---|
| 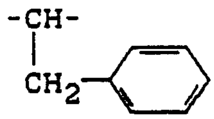 | H | - | 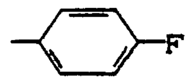 |
| 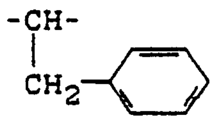 | H | - | 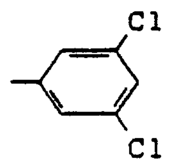 |
| 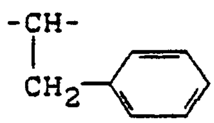 | H | - | 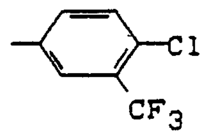 |
| 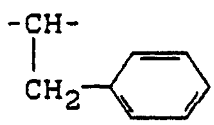 | H | - | 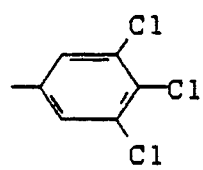 |
| 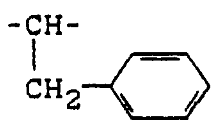 | H | - | 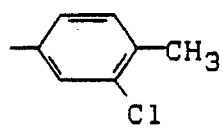 |
| 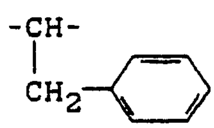 | H | - | 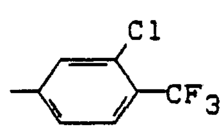 |

| $A^1$ | R | $(A^2)_n$ | Ar |
|---|---|---|---|
| 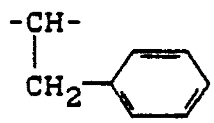 | H | $-CH_2-$ | 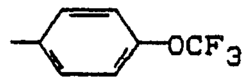 |
| 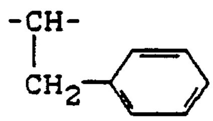 | H | $-CH_2-$ | 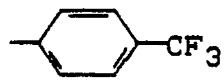 |
| 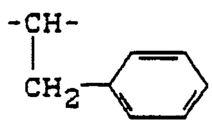 | H | $-CH_2-$ | 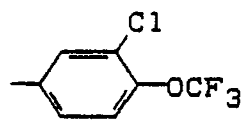 |
| 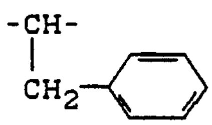 | H | $-(CH_2)_2-$ | 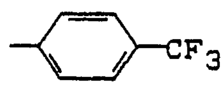 |
| 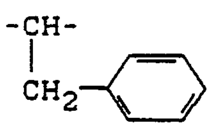 | H | $-(CH_2)_2-$ | 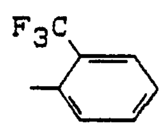 |
| 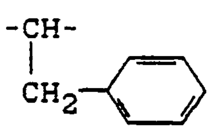 | H | $-(CH_2)_2-$ | 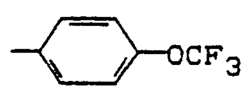 |

| $A^1$ | R | $(A^2)_n$ | Ar |
|---|---|---|---|
| 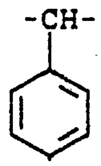 | H | - | 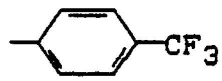 |
| 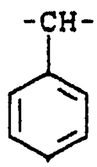 | H | - | 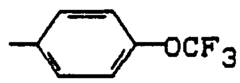 |
| 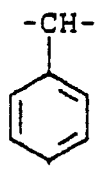 | H | - | 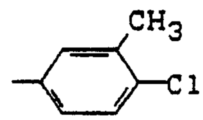 |
| 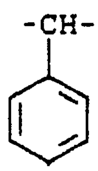 | H | - | 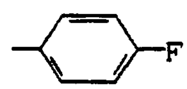 |
| 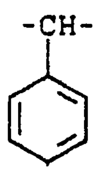 | H | - | 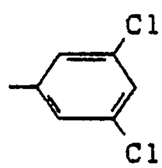 |

| $A^1$ | R | $(A^2)_n$ | Ar |
|---|---|---|---|
| 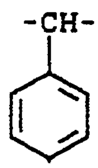 | H | - | 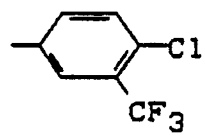 |
| 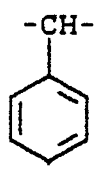 | H | - | 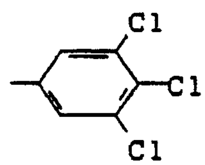 |
| 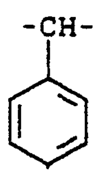 | H | - | 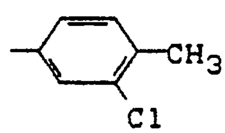 |
| 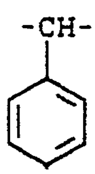 | H | - | 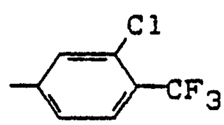 |
| 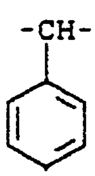 | H | $-CH_2-$ | 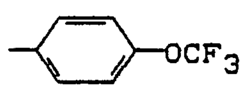 |

| $A^1$ | R | $(A^2)_n$ | Ar |
|---|---|---|---|
| 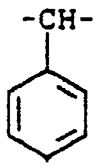 | H | $-CH_2-$ | 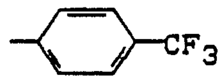 |
| 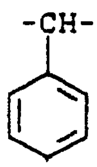 | H | $-CH_2-$ | 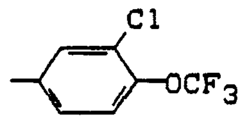 |
| 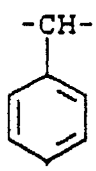 | H | $-(CH_2)_2-$ | 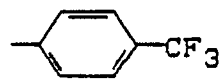 |
| 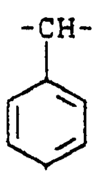 | H | $-(CH_2)_2-$ | 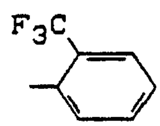 |
| 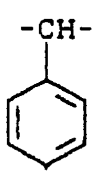 | H | $-(CH_2)_2-$ | 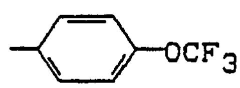 |

| $A^1$ | R | $(A^2)_n$ | Ar |
|---|---|---|---|
| $-(CH_2)_4-$ | H | - | 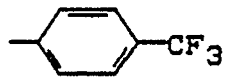 |
| $-(CH_2)_4-$ | H | - | 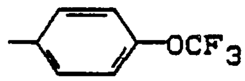 |
| $-(CH_2)_4-$ | H | - | 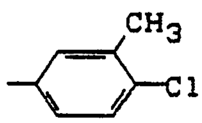 |
| $-(CH_2)_4-$ | H | - | 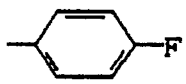 |
| $-(CH_2)_4-$ | H | - | 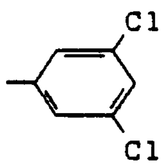 |
| $-(CH_2)_4-$ | H | - | 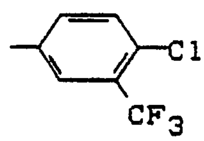 |
| $-(CH_2)_4-$ | H | - | 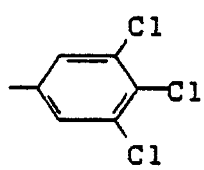 |

| $A^1$ | R | $(A^2)_n$ | Ar |
|---|---|---|---|
| $-(CH_2)_4-$ | H | - | 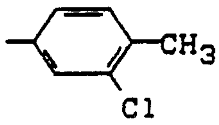 |
| $-(CH_2)_4-$ | H | - | 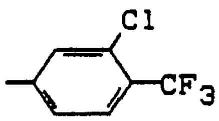 |
| $-(CH_2)_4-$ | H | $-CH_2-$ | 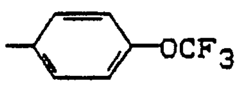 |
| $-(CH_2)_4-$ | H | $-CH_2-$ | 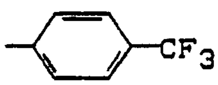 |
| $-(CH_2)_4-$ | H | $-CH_2-$ | 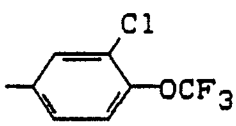 |
| $-(CH_2)_4-$ | H | $-(CH_2)_2-$ | 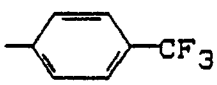 |
| $-(CH_2)_4-$ | H | $-(CH_2)_2-$ | 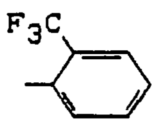 |
| $-(CH_2)_4-$ | H | $-(CH_2)_2-$ | 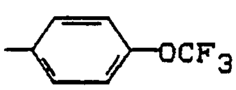 |

| $A^1$ | R | $(A^2)_n$ | Ar |
|---|---|---|---|
| $-(CH_2)_5-$ | H | - | 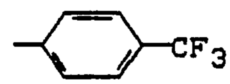 |
| $-(CH_2)_5-$ | H | - | 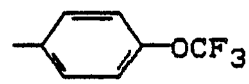 |
| $-(CH_2)_5-$ | H | - | 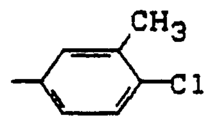 |
| $-(CH_2)_5-$ | H | - | 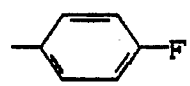 |
| $-(CH_2)_5-$ | H | - | 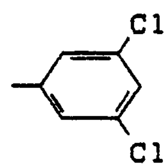 |
| $-(CH_2)_5-$ | H | - | 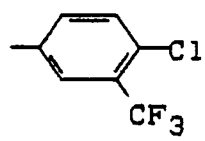 |
| $-(CH_2)_5-$ | H | - | 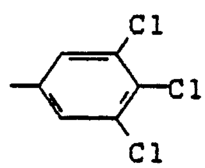 |
| $-(CH_2)_5-$ | H | - | 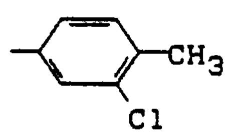 |

| $A^1$ | R | $(A^2)_n$ | Ar |
|---|---|---|---|
| $-(CH_2)_5-$ | H | - | 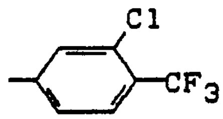 |
| $-(CH_2)_5-$ | H | $-CH_2-$ | 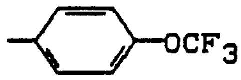 |
| $-(CH_2)_5-$ | H | $-CH_2-$ | 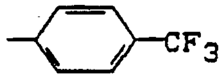 |
| $-(CH_2)_5-$ | H | $-CH_2-$ | 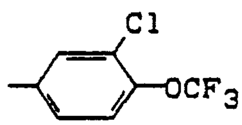 |
| $-(CH_2)_5-$ | H | $-(CH_2)_2-$ | 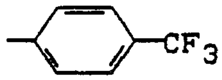 |
| $-(CH_2)_5-$ | H | $-(CH_2)_2-$ | 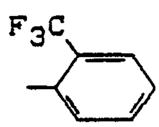 |
| $-(CH_2)_5-$ | H | $-(CH_2)_2-$ | 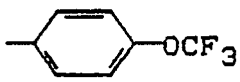 |
| $-(CH_2)_6-$ | H | - | 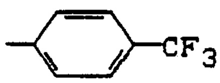 |

| $A^1$ | R | $(A^2)_n$ | Ar |
|---|---|---|---|
| $-(CH_2)_6-$ | H | - | 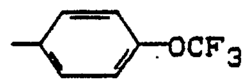 |
| $-(CH_2)_6-$ | H | - | 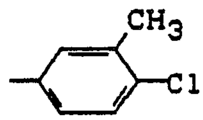 |
| $-(CH_2)_6-$ | H | - | 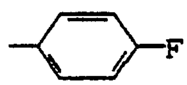 |
| $-(CH_2)_6-$ | H | - | 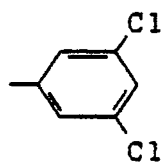 |
| $-(CH_2)_6-$ | H | - | 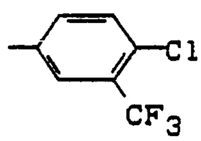 |
| $-(CH_2)_6-$ | H | - | 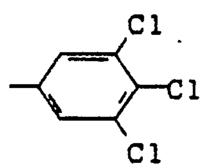 |
| $-(CH_2)_6-$ | H | - | 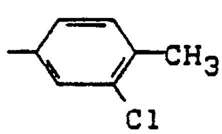 |

| $A^1$ | R | $(A^2)_n$ | Ar |
|---|---|---|---|
| $-(CH_2)_6-$ | H | - | 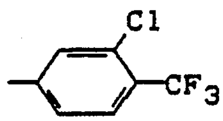 |
| $-(CH_2)_6-$ | H | $-CH_2-$ | 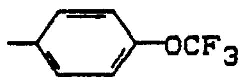 |
| $-(CH_2)_6-$ | H | $-CH_2-$ | 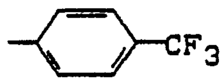 |
| $-(CH_2)_6-$ | H | $-CH_2-$ | 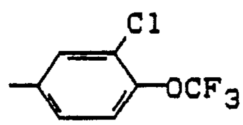 |
| $-(CH_2)_6-$ | H | $-(CH_2)_2-$ | 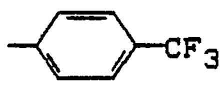 |
| $-(CH_2)_6-$ | H | $-(CH_2)_2-$ | 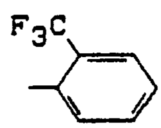 |
| $-(CH_2)_6-$ | H | $-(CH_2)_2-$ | 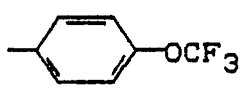 |
| $-CH(CH_3)-CH_2-$ | H | - | 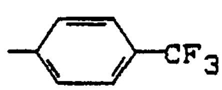 |

| $A^1$ | R | $(A^2)_n$ | Ar |
|---|---|---|---|
| $-CH-CH_2-$<br>$\vert$<br>$CH_3$ | H | - | 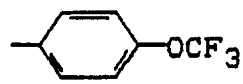 |
| $-CH-CH_2-$<br>$\vert$<br>$CH_3$ | H | - | 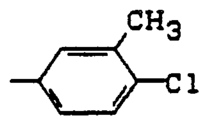 |
| $-CH-CH_2-$<br>$\vert$<br>$CH_3$ | H | - | 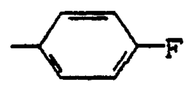 |
| $-CH-CH_2-$<br>$\vert$<br>$CH_3$ | H | - | 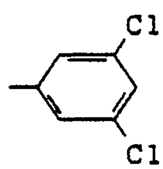 |
| $-CH-CH_2-$<br>$\vert$<br>$CH_3$ | H | - | 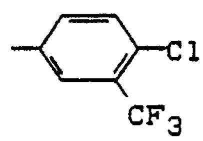 |
| $-CH-CH_2-$<br>$\vert$<br>$CH_3$ | H | - | 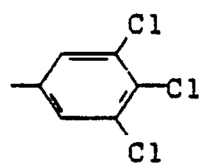 |
| $-CH-CH_2-$<br>$\vert$<br>$CH_3$ | H | - | 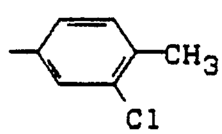 |

| $A^1$ | R | $(A^2)_n$ | Ar |
|---|---|---|---|
| $-CH-CH_2-$<br>\|<br>$CH_3$ | H | - | 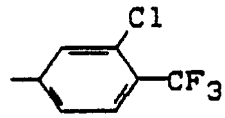 |
| $-CH-CH_2-$<br>\|<br>$CH_3$ | H | $-CH_2-$ | 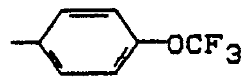 |
| $-CH-CH_2-$<br>\|<br>$CH_3$ | H | $-CH_2-$ | 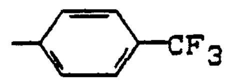 |
| $-CH-CH_2-$<br>\|<br>$CH_3$ | H | $-CH_2-$ | 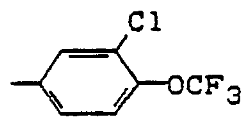 |
| $-CH-CH_2-$<br>\|<br>$CH_3$ | H | $-(CH_2)_2-$ | 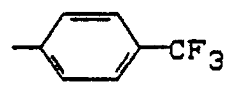 |
| $-CH-CH_2-$<br>\|<br>$CH_3$ | H | $-(CH_2)_2-$ | 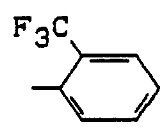 |
| $-CH-CH_2-$<br>\|<br>$CH_3$ | H | $-(CH_2)_2-$ | 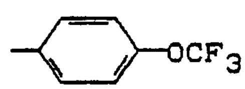 |

| $A^1$ | R | $(A^2)_n$ | Ar |
|---|---|---|---|
| $-(CH_2)_5-$ | H | - | 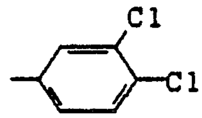 |
| $-(CH_2)_5-$ | H | - | 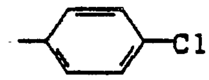 |
| $-(CH_2)_5-$ | H | - | 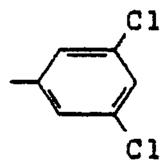 |
| $-(CH_2)_5-$ | H | - | 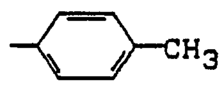 |
| $-(CH_2)_5-$ | H | - | 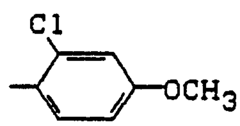 |
| $-(CH_2)_5-$ | H | $-CH_2-$ | 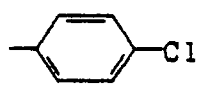 |
| $-(CH_2)_5-$ | H | $-CH_2-$ | 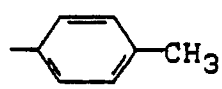 |
| $-(CH_2)_5-$ | H | $-(CH_2)_2-$ | 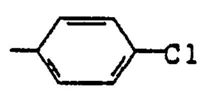 |

| $A^1$ | R | $(A^2)_n$ | Ar |
|---|---|---|---|
| $-(CH_2)_5-$ | H | $-(CH_2)_2-$ | 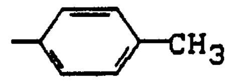 |
| $-(CH_2)_4-$ | H | - | 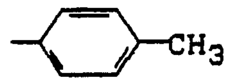 |
| $-(CH_2)_4-$ | H | - | 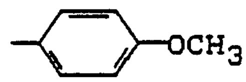 |
| $-(CH_2)_4-$ | H | - | 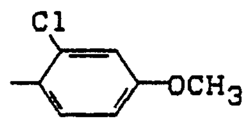 |
| $-(CH_2)_4-$ | H | $-(CH_2)_2-$ | 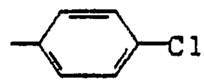 |
| $-(CH_2)_4-$ | H | $-(CH_2)_2-$ | 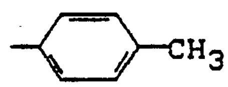 |
| $-(CH_2)_6-$ | H | - | 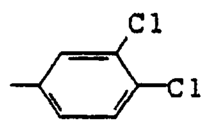 |
| $-(CH_2)_6-$ | H | - | 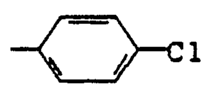 |

| $A^1$ | R | $(A^2)_n$ | Ar |
|---|---|---|---|
| $-(CH_2)_6-$ | H | - | 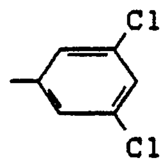 |
| $-(CH_2)_6-$ | H | - | 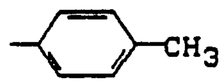 |
| $-(CH_2)_6-$ | H | - | 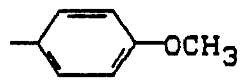 |
| $-(CH_2)_6-$ | H | - | 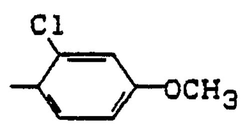 |
| $-(CH_2)_6-$ | H | $-CH_2-$ | 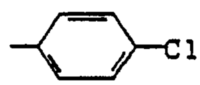 |
| $-(CH_2)_6-$ | H | $-CH_2-$ | 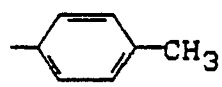 |
| $-(CH_2)_6-$ | H | $-(CH_2)_2-$ | 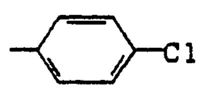 |
| $-(CH_2)_6-$ | H | $-(CH_2)_2-$ | 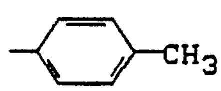 |

| $A^1$ | R | $(A^2)_n$ | Ar |
|---|---|---|---|
| $-(CH_2)_4-$ | H | $-CH_2-$ | 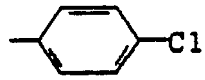 |
| $-(CH_2)_4-$ | H | $-CH_2-$ | 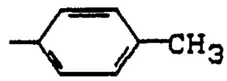 |
| $-CH-CH_2-$<br>$CH_3$ | H | - | 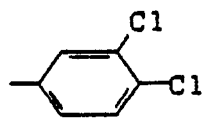 |
| $-CH-CH_2-$<br>$CH_3$ | H | - | 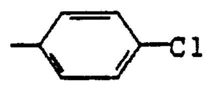 |
| $-CH-CH_2-$<br>$CH_3$ | H | - | 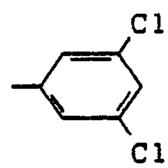 |
| $-CH-CH_2-$<br>$CH_3$ | H | - | 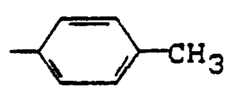 |
| $-CH-CH_2-$<br>$CH_3$ | H | - | 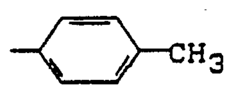 |

| $A^1$ | R | $(A^2)_n$ | Ar |
|---|---|---|---|
| $-CH-CH_2-$<br>$CH_3$ | H | - | 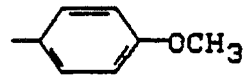 |
| $-CH-CH_2-$<br>$CH_3$ | H | - | 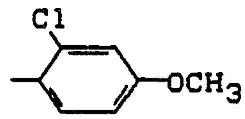 |
| $-CH-CH_2-$<br>$CH_3$ | H | $-CH_2-$ | 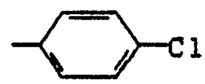 |
| $-CH-CH_2-$<br>$CH_3$ | H | - | 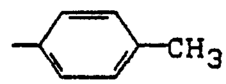 |
| $-CH-CH_2-$<br>$CH_3$ | H | $-(CH_2)_2-$ | 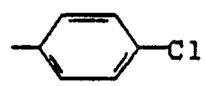 |
| $-CH-CH_2-$<br>$CH_3$ | H | $-(CH_2)_2-$ | 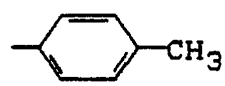 |

Verwendet man beispielsweise 2-(Dichlormaleinimido)essigsäurechlorid und 3,4,5-Trichloranilin als Ausgangsstoffe, so läßt sich der Reaktionsablauf des erfindungsgemäßen Verfahrens durch das folgende Formelschema darstellen:

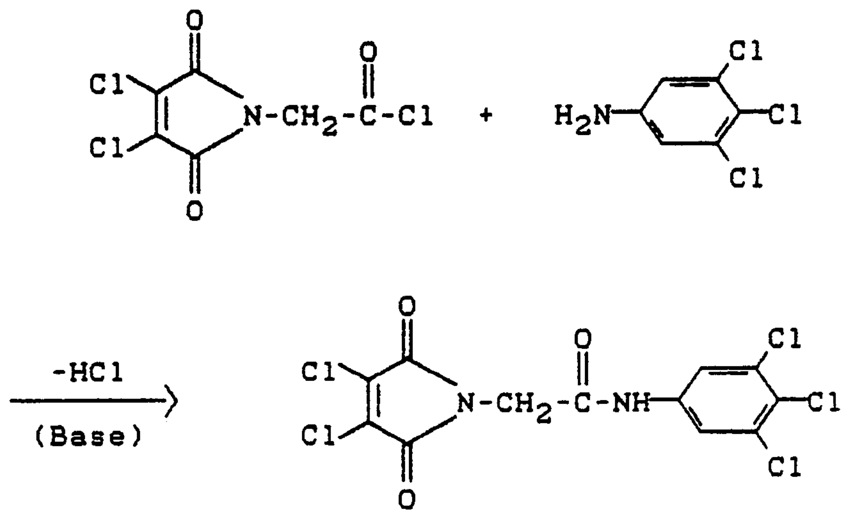


Die zur Durchführung des erfindungsgemäßen Verfahrens als Ausgangsstoffe benötigten Dichlormaleinimidocarbonsäurehalogenide sind durch die Formel (II) allgemein definiert. In dieser Formel (II) steht $A^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Hal steht vorzugsweise für Chlor oder Brom.

Die Dichlormaleinimidocarbonsäurehalogenide der Formel (II) sind teilweise bekannt (vgl. CH 617 320 vom 30.05.1980). Man erhält sie in Analogie zu bekannten Verfahren, wenn man Dichlormaleinsäureanhydrid der Formel (IV),

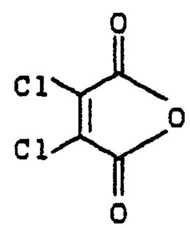

(IV)

mit Aminosäuren der Formel (V),

$H_2N\text{-}A^1\text{-}COOH$ (V)

in welcher

$A^1$ die oben angegebene Bedeutung hat,

gegebenenfalls in Gegenwart eines Verdünnungsmittels, wie beispielsweise Eisessig, bei Temperaturen zwischen 30 °C und 150 °C umsetzt und die so erhältlichen Dichlormaleimidocarbonsäuren der Formel (VI),

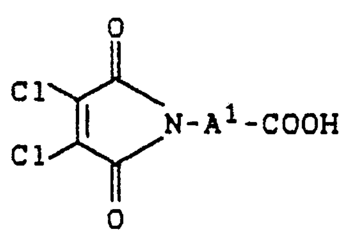

(VI)

in welcher

$A^1$ die oben angegebene Bedeutung hat,

in üblicher Art und Weise mit Halogenierungsmitteln, wie beispielsweise Phosphorpentachlorid, Phosphortribromid, Phosphoroxychlorid oder Thionylchlorid, bei Temperaturen zwischen 30 °C und 150 °C umsetzt.

Dichlormaleinimidocarbonsäuren der Formel (VI) sind größtenteils bekannt (vgl. J. Prakt. Chem. 327,

857 -864 [1985]).

Dichlormaleinsäureanhydrid der Formel (IV) ist bekannt (vgl. z.B. DE-OS 12 90 555 oder US-PS 32 97 722).

Aminosäuren der Formel (V) sind allgemein bekannte Verbindungen der organischen Chemie.

Die zur Durchführung des erfindungsgemäßen Verfahrens weiterhin als Ausgangsstoffe benötigten Amine sind durch die Formel (III) allgemein definiert. In dieser Formel (III) stehen R, Ar, $A^2$ und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die Amine der Formel (III) sind ebenfalls allgemein bekannte Verbindungen der organischen Chemie.

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren wird vorzugsweise in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens setzt man pro Mol an Dichlormaleimidocarbonsäurehalogenid der Formel (II) in allgemeinen 1.0 bis 2.5 Mol, vorzugsweise 1.0 bis 1.5 Mol an Amin der Formel (III) und gegebenenfalls 1.0 bis 2.5 Mol, vorzugsweise 1.0 bis 1.5 Mol an Säurebindemittel ein. Die Reaktionsdurchführung, Aufarbeitung und Isolierung der Reaktionsprodukte erfolgt nach allgemein üblichen Methoden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide und Bakterizide einsetzbar.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;

Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen den Erreger des falschen Rebenmehltaus (Plasmopara viticola) oder gegen den Erreger des Apfelschorfes (Venturia inaequalis) oder zur Bekämpfung von samenbürtigen Krankheitserregern der Sojabohne (beispielsweise Rhizoctonia solani, Fusarium sp., Pythium sp.), oder zur Bekämpfung von Reiskrankheiten, wie beispielsweise gegen den Erreger der Reisfleckenkrankheit (Pyricularia oryzae) einsetzen. Die systemischen Eigenschaften der erfindungsgemäßen Verbindungen ermöglichen eine Anwendung als Saatgutbeizmittel.

Darüberhinaus besitzen die erfindungsgemäßen Wirkstoffe auch bakterizide Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenak tiven Mitteln, als Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeitten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haft mittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1

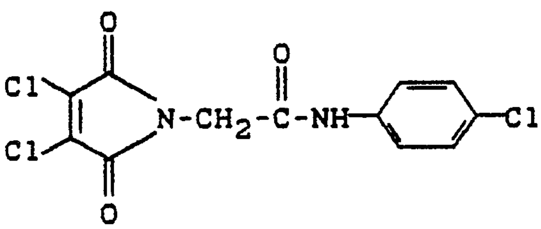

Zu 6,0 g (0,025 Mol) 2-Dichlormaleinimidoacetylchlorid in 60 ml Dioxan gibt man tropfenweise unter Rühren eine Mischung aus 3,5 g (0,027 Mol) p-Chloranilin und 3,45 ml (0,025 Mol) Triethylamin in 40 ml Dioxan, wobei die Temperatur der Reaktionsmischung auf 40 °C ansteigt. Nach beendeter Zugabe rührt man 4 Stunden bei Raumtemperatur, gießt dann die Mischung in 1N Salzsäure, saugt den entstandenen Niederschlag ab, wäscht ihn mit gesättigter wässriger Natriumhydrogencarbonatlösung und Wasser und trocknet ihn im Vakuum bei 50 °C.

Man erhält 7,1 g (96 % der Theorie) an 2-(Dichlormaleinimido)-N-(4-chlorphenyl)-acetamid vom Schmelzpunkt 212 °C - 214 °C.

## Herstellung der Ausgangsverbindung

### Beispiel II-1

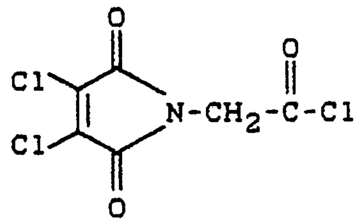

Zu 70 g (0.31 Mol) 2-(Dichlormaleinimido)-essigsäure gibt man 60 ml (0.81 Mol) Thionylchlorid, erhitzt für 16 Stunden auf Rückflußtemperatur, engt dann im Vakuum ein, nimmt den Rückstand in absolutem Toluol auf und entfernt das Lösungsmittel und restliches Thionylchlorid im Vakuum.

Man erhält 75,1 g (99 % der Theorie) an 2-(Dichlormaleinimido)-acetylchlorid vom Schmelzpunkt 102 °C -103 °C.

Beispiel VI-1

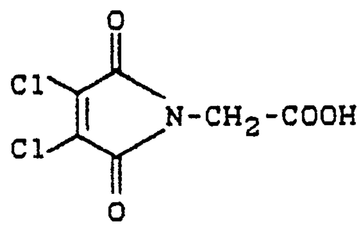

Zu 167 g (1 Mol) Dichlormaleinsäureanhydrid in 1000 ml Eisessig gibt man eine Suspension aus 75 g (1.0 Mol) Glycin in 400 ml Eisessig, erhitzt für eine Stunde auf Rückflußtemperatur, kühlt ab, saugt den ausgefallenen Niederschlag ab, wäscht mit 2,5 l Wasser nach und trocknet im Vakuum bei 50 °C.

Man erhält 139,9 g (63 % der Theorie) an 2-(Dichlormaleinimido)-essigsäure vom Schmelzpunkt 191 °C -192 °C.

In entsprechender Weise und gemäß den allgemeinen Angaben zur Herstellung erhält man die folgenden N-substituierten Dichlormaleinimide der allgemeinen Formel (I):

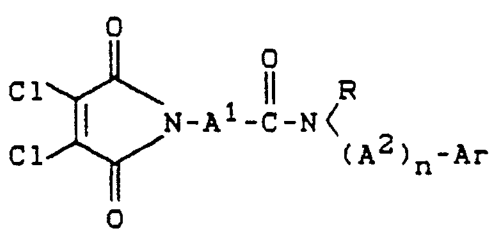

(I)

| Bsp. Nr. | $A^1$ | R | $(A^2)_n$ | Ar | Schmelzpunkt |
|---|---|---|---|---|---|
| 2 | $-CH_2-$ | H | - | 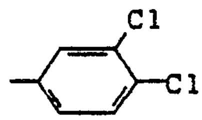 | 218-220° C |
| 3 | $-CH_2-$ | H | - | 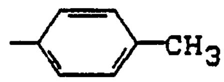 | 213-215° C |
| 4 | $-CH_2-$ | H | - | 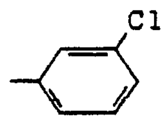 | 193-194° C |
| 5 | $-CH_2-$ | H | - | 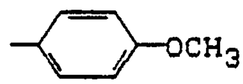 | 211-213° C |
| 6 | $-CH_2-$ | H | - | 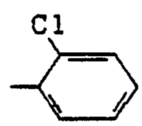 | 186-188° C |
| 7 | $-CH_2-$ | H | - | 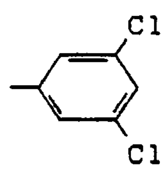 | 233-235° C |
| 8 | $-CH_2-$ | H | - | 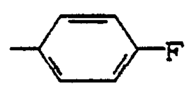 | 177-178° C |

| Bsp. Nr. | $A^1$ | R | $(A^2)_n$ | Ar | Schmelzpunkt |
|---|---|---|---|---|---|
| 9 | -CH-<br>$CH(CH_3)_2$ | H | - | 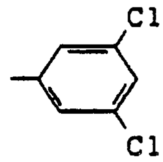 | 169-171° C |
| 10 | -CH-<br>$CH(CH_3)_2$ | H | - | 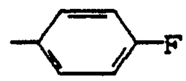 | $^1$H-NMR*): 4,35-4,45 (1H) |
| 11 | -CH-<br>$CH(CH_3)_2$ | H | - | 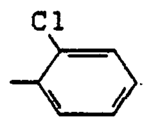 | 89-91° C |
| 12 | -CH-<br>$CH(CH_3)_2$ | H | - | 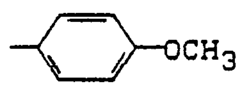 | 116-118° C |
| 13 | -CH-<br>$CH(CH_3)_2$ | H | - | 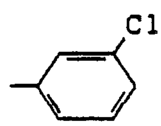 | 140-141° C |
| 14 | -CH-<br>$CH(CH_3)_2$ | H | - | 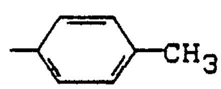 | 70-71° C |
| 15 | -CH-<br>$CH(CH_3)_2$ | H | - | 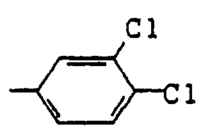 | $^1$H-NMR*): 4,3-4,4 (1H) |

| Bsp. Nr. | $A^1$ | R | $(A^2)_n$ | Ar | Schmelzpunkt |
|---|---|---|---|---|---|
| 16 | $-CH(CH(CH_3)_2)-$ | H | - | 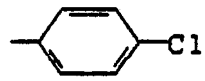 | 135-138° C |
| 17 | $-CH(CH(CH_3)_2)-$ | H | - | 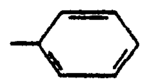 | 146-148° C |
| 18 | $-CH_2-$ | $CH_3$ | - | 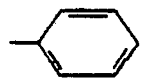 | 193-195° C |
| 19 | $-CH_2-$ | H | - | 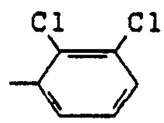 | 216° C |
| 20 | $-CH_2-$ | H | - | 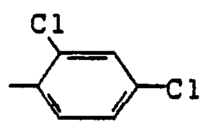 | 208° C |
| 21 | $-CH_2-$ | H | - | 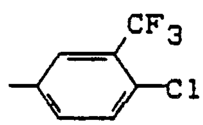 | 174-177° C |
| 22 | $-CH_2-$ | H | - | 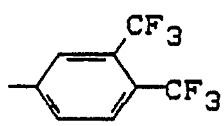 | 158° C |

| Bsp. Nr. | $A^1$ | R | $(A^2)_n$ | Ar | Schmelzpunkt |
|---|---|---|---|---|---|
| 23 | $-CH_2-$ | H | - | 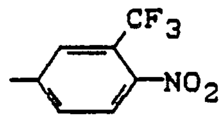 | 189-192° C |
| 24 | $-CH_2-$ | H | - | 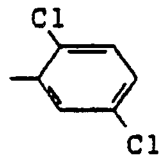 | 230° C |
| 25 | $-CH_2-$ | H | - | 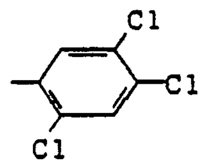 | 220° C |
| 26 | $-CH_2-$ | H | - | 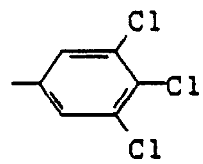 | 237° C |
| 27 | $-CH_2-$ | H | - | 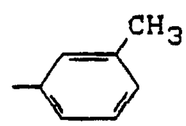 | 222° C |
| 28 | $-CH_2-$ | H | - | 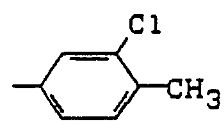 | 213° C |
| 29 | $-CH_2-$ | H | $-CH_2-$ | 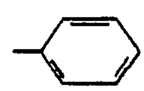 | 173-175° C |

| Bsp. Nr. | $A^1$ | R | $(A^2)_n$ | Ar | Schmelzpunkt |
|---|---|---|---|---|---|
| 30 | $-CH_2-$ | H | $-CH_2-$ | 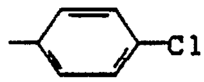 | 192-194° C |
| 31 | $-CH_2-$ | H | - | 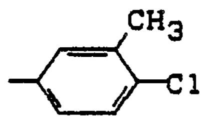 | 217-218° C |
| 32 | $-CH_2-$ | H | $-CH_2-$ | 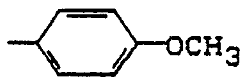 | 161-163° C |
| 33 | $-CH_2-$ | H | $-CH-$<br>$CH_3$ | 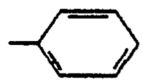 | 172-173° C |
| 34 | $-CH_2-$ | H | $-(CH_2)_2-$ | 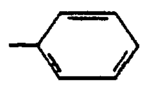 | 170-171° C |
| 35 | $-CH_2-$ | H | - | 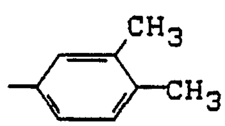 | >220° C |
| 36 | $-CH-$<br>$CH_2-C_6H_5$ | H | - | 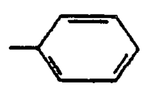 | 183-184° C (L-Form) |
| 37 | $-CH-$<br>$CH_2-C_6H_5$ | H | - | 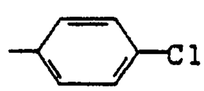 | 235-236° C (L-Form) |

| Bsp. Nr. | $A^1$ | R | $(A^2)_n$ | Ar | Schmelzpunkt |
|---|---|---|---|---|---|
| 38 | $-CH(CH_2-C_6H_5)-$ | H | - | 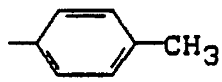 | 183-184° C (L-Form) |
| 39 | $-CH(CH_2-C_6H_5)-$ | H | - | 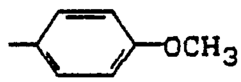 | 178-180° C (L-Form) |
| 40 | $-CH(CH_2-C_6H_5)-$ | H | - | 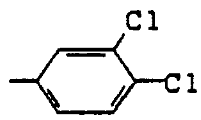 | 199-200 (L-Form) |
| 41 | $-CH(CH_2-C_6H_5)-$ | H | $-CH_2-$ | 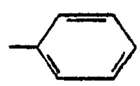 | 163-164° C (L-Form) |
| 42 | $-CH(CH_2-C_6H_5)-$ | H | $-CH_2-$ | 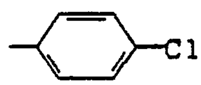 | 143-144° C (L-Form) |
| 43 | $-CH_2-$ | H | - | 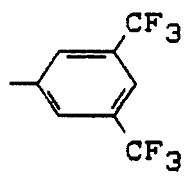 | 206-208° C |
| 44 | $-CH_2-$ | H | - | 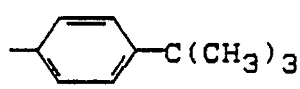 | 206-207° C |

| Bsp. Nr. | $A^1$ | R | $(A^2)_n$ | Ar | Schmelzpunkt |
|---|---|---|---|---|---|
| 45 | $-CH_2-$ | H | - | 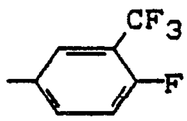 | 194-196° C |
| 46 | $-CH_2-$ | H | - | 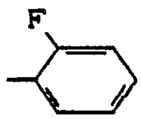 | 180-182° C |
| 47 | $-CH_2-$ | H | - | 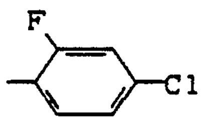 | 204-205° C |
| 48 | $-CH_2-$ | H | - | 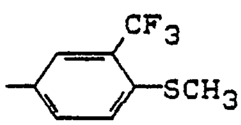 | 180-182° C |
| 49 | $-CH_2-$ | H | - | 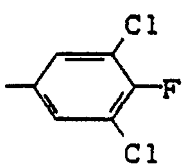 | 208-210° C |
| 50 | $-CH_2-$ | H | - | 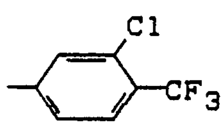 | 189-190° C |
| 51 | $-CH_2-$ | H | - | 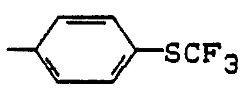 | 180-182° C |

| Bsp. Nr. | $A^1$ | R | $(A^2)_n$ | Ar | Schmelzpunkt |
|---|---|---|---|---|---|
| 52 | $-CH_2-$ | H | - | 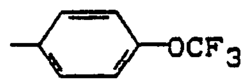 | 174-176° C |
| 53 | $-CH_2-$ | H | - | 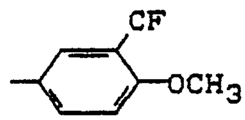 | 180-182° C |
| 54 | $-CH_2-$ | H | - | 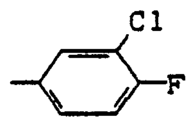 | 194° C |
| 55 | $-CH_2-$ | H | - | 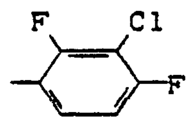 | 189-190° C |
| 56 | $-CH_2-$ | H | - | 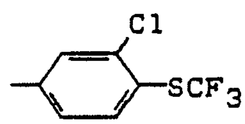 | 152° C |
| 57 | $-CH_2-$ | H | $-CH_2-$ | 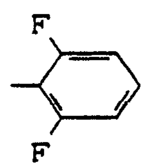 | 199° C |
| 58 | $-CH_2-$ | H | $-CH_2-$ | 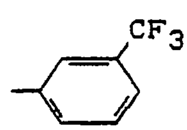 | 172° C |

| Bsp. Nr. | $A^1$ | R | $(A^2)_n$ | Ar | Schmelzpunkt |
|---|---|---|---|---|---|
| 59 | $-CH_2-$ | H | - | 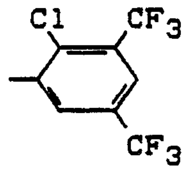 | 230° C |
| 60 | $-CH_2-$ | H | $-CH_2-$ | 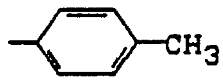 | 181° C |
| 61 | $-CH_2-$ | H | $-CH_2-$ | 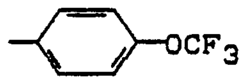 | 184° C |
| 62 | $-CH_2-$ | H | $-CH_2-$ | 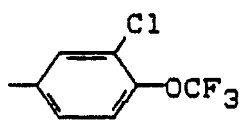 | 210° C |
| 63 | $-CH_2-$ | H | $-(CH_2)_2-$ | 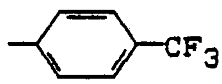 | 179° C |
| 64 | $-CH_2-$ | H | $-(CH_2)_2-$ | 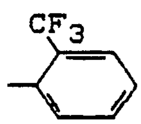 | 185° C |
| 65 | $-CH_2-$ | H | $-(CH_2)_2-$ | 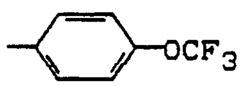 | 160-162° C |
| 66 | $-CH(CH_3)-$ | H | $-CH_2-$ | 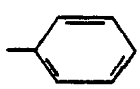 | 96° C (L-Form) |

| Bsp. Nr. | $A^1$ | R | $(A^2)_n$ | Ar | Schmelzpunkt |
|---|---|---|---|---|---|
| 67 | $-CH-$<br>$CH_3$ | H | $-CH_2-$ | 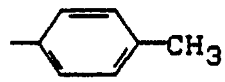 | 142-143° C (L-Form) |
| 68 | $-CH-$<br>$CH_3$ | H | - | 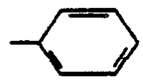 | 183-184° C |
| 69 | $-CH-$<br>$CH_3$ | H | - | 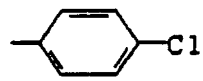 | 181° C (L-Form) |
| 70 | $-CH-$<br>$CH_3$ | H | - | 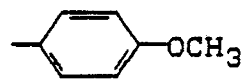 | 159-162° C (L-Form) |
| 71 | $-CH-$<br>$CH_3$ | H | - | 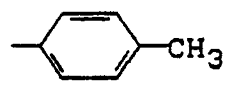 | 194-195° C (L-Form) |
| 72 | $-CH-$<br>$CH_3$ | H | - | 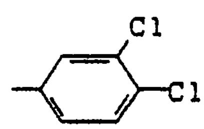 | 65° C (L-Form) |
| 73 | $-CH_2-CH_2-$ | H | - | 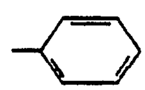 | 197-198° C |

| Bsp. Nr. | $A^1$ | R | $(A^2)_n$ | Ar | Schmelzpunkt |
|---|---|---|---|---|---|
| 74 | $-CH_2-CH_2-$ | H | - | 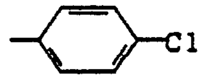 | 208-210° C |
| 75 | $-CH_2-CH_2-$ | H | - | 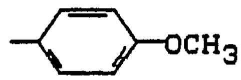 | 188-189° C |
| 76 | $-CH_2-CH_2-$ | H | - | 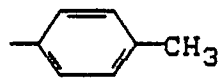 | 190-191° C |
| 77 | $-CH_2-CH_2-$ | H | - | 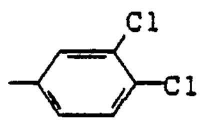 | 191° C |
| 78 | $-CH_2-CH_2-$ | H | $-CH_2-$ | 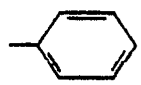 | 171-172° C |
| 79 | $-CH_2-CH_2-$ | H | $-CH_2-$ | 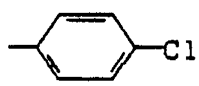 | 177-178° C |
| 80 | $-CH(CH_3)-$ | H | - | 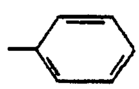 | 173° C (D,L-Form) |
| 81 | $-CH(CH_3)-$ | H | - | 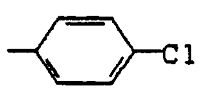 | 168-170° C (D,L-Form) |

| Bsp. Nr. | $A^1$ | R | $(A^2)_n$ | Ar | Schmelzpunkt |
|---|---|---|---|---|---|
| 82 | $-(CH_2)_3-$ | H | - | 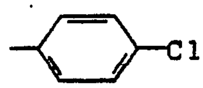 | 160-162° C |
| 83 | $-CH_2-$ | $CH_3$ | - | 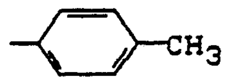 | 121-122° C |
| 84 | $-(CH_2)_3-$ | H | - | 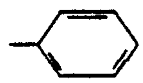 | 139-140° C |
| 85 | $-(CH_2)_3-$ | H | - | 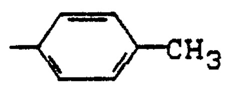 | 166° C |
| 86 | $-(CH_2)_3-$ | H | - | 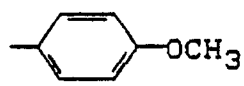 | 158° C |
| 87 | $-(CH_2)_3-$ | H | - | 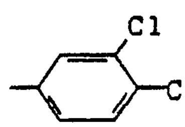 | 156-158° C |
| 88 | $-(CH_2)_3-$ | H | $-CH_2-$ | 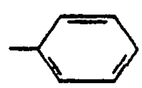 | 150-152° C |
| 89 | $-(CH_2)_3-$ | H | $-CH_2-$ | 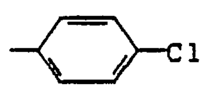 | 186-187° C |

| Bsp. Nr. | $A^1$ | R | $(A^2)_n$ | Ar | Schmelzpunkt |
|---|---|---|---|---|---|
| 90 | 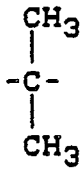 | H | - | 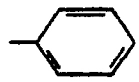 | $201-203^{\circ}$ C |
| 91 | 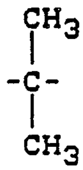 | H | - | 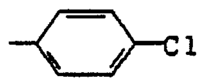 | $199-200^{\circ}$ C |
| 92 | 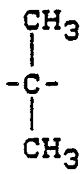 | H | - | 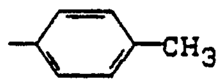 | $198^{\circ}$ C |
| 93 | 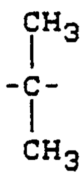 | H | - | 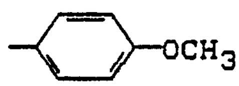 | $161-163^{\circ}$ C |
| 94 | 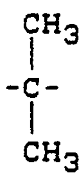 | H | - | 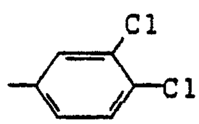 | $74-76^{\circ}$ C |
| 95 | 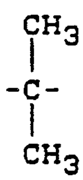 | H | $-CH_2-$ | 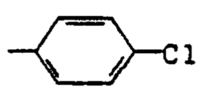 | $164^{\circ}$ C |

| Bsp. Nr. | $A^1$ | R | $(A^2)_n$ | Ar | Schmelzpunkt |
|---|---|---|---|---|---|
| 96 | $-CH-$<br>\|<br>$CH_2-C_6H_5$ | H | - | 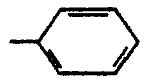 | 211-212° C (D,L-Form) |
| 97 | $-CH-$<br>\|<br>$CH_2-C_6H_5$ | H | - | 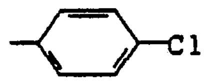 | 219° C (D,L-Form) |
| 98 | $-CH-$<br>\|<br>$CH_2-C_6H_5$ | H | - | 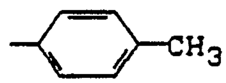 | 181° C (D,L-Form) |
| 99 | $-CH-$<br>\|<br>$CH_2-C_6H_5$ | H | - | 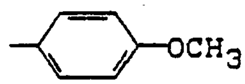 | 182-183° C (D,L-Form) |
| 100 | $-CH-$<br>\|<br>$CH_2-C_6H_5$ | H | - | 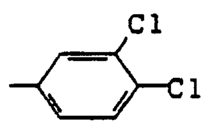 | 197° C (D,L-Form) |
| 101 | $-CH-$<br>\|<br>$CH_2-C_6H_5$ | H | $-CH_2-$ | 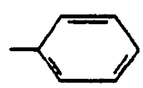 | 186-187° C (D,L-Form) |
| 102 | $-CH-$<br>\|<br>$CH_2-C_6H_5$ | H | $-CH_2-$ | 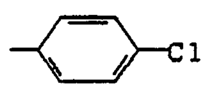 | 158° C (D,L-Form) |

| Bsp. Nr. | $A^1$ | R | $(A^2)_n$ | Ar | Schmelzpunkt |
|---|---|---|---|---|---|
| 103 | 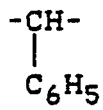 | H | - | 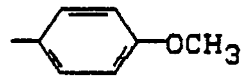 | 157-158° C |
| 104 | 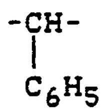 | H | - | 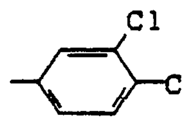 | 144-146° C |
| 105 | 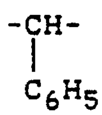 | H | $-CH_2-$ | 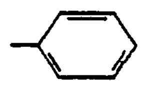 | 145° C |
| 106 | 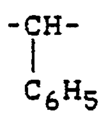 | H | $-CH_2-$ | 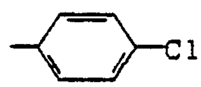 | 190-191° C |
| 107 | $-CH_2-$ | H | 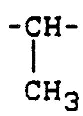 | 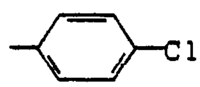 | 175° C |
| 108 | $-CH_2-CH_2-$ | H | - | 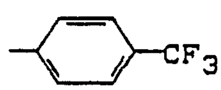 | 208-210° C |
| 109 | $-CH_2-CH_2-$ | H | $-CH_2-CH_2-$ | 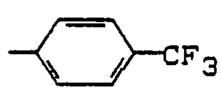 | 162-164° C |

| Bsp. Nr. | $A^1$ | R | $(A^2)_n$ | Ar | Schmelzpunkt |
|---|---|---|---|---|---|
| 110 | $-CH_2-CH_2-$ | H | $-CH_2-CH_2-$ | 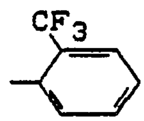 | 131° C |
| 111 | $-CH_2-CH_2-$ | H | $-CH_2-CH_2-$ | 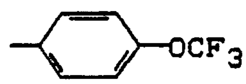 | 159-161° C |
| 112 | $-CH_2-CH_2-$ | H | - | 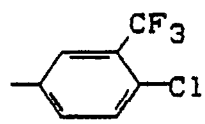 | 130-131° C |
| 113 | $-CH_2-CH_2-$ | H | $-CH_2-$ | 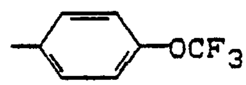 | 160° C |
| 114 | $-CH_2-CH_2-$ | H | $-CH_2-$ | 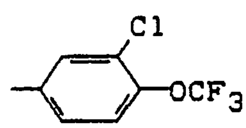 | 134-137° C |
| 115 | $-CH_2-CH_2-$ | H | - | 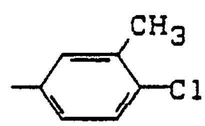 | 194° C |
| 116 | $-CH_2-CH_2-$ | H | - | 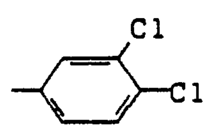 | 181-183° C |

| Bsp. Nr. | $A^1$ | R | $(A^2)_n$ | Ar | Schmelzpunkt |
|---|---|---|---|---|---|
| 117 | $-CH_2-CH_2-$ | H | - | 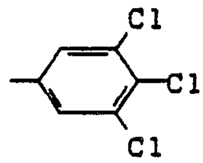 | 208° C |
| 118 | $-CH_2-CH_2-$ | H | - | 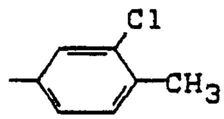 | 179° C |
| 119 | $-(CH_2)_3-$ | H | - | 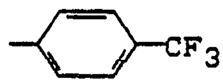 | 132-134° C |
| 120 | $-(CH_2)_3-$ | H | - | 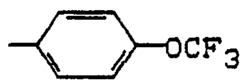 | 143° C |
| 121 | $-(CH_2)_3-$ | H | - | 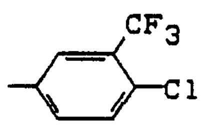 | 80-82° C |
| 122 | $-(CH_2)_3-$ | H | $-CH_2-$ | 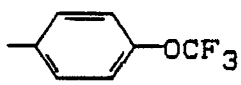 | 126 °C |
| 123 | $-(CH_2)_3-$ | H | $-CH_2-$ | 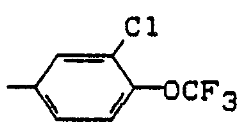 | 118-120° C |

| Bsp. Nr. | $A^1$ | R | $(A^2)_n$ | Ar | Schmelzpunkt |
|---|---|---|---|---|---|
| 124 | $-(CH_2)_3-$ | H | - | 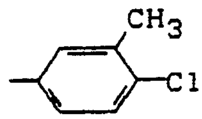 | 166-167° C |
| 125 | $-CH_2-CH_2-$ | H | - | 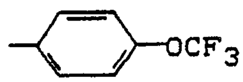 | 183° C |
| 126 | $-CH_2-CH_2-$ | H | - | 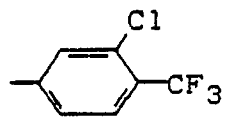 | 199-202° C |
| 127 | $-CH_2-CH_2-CH_2-$ | H | - | 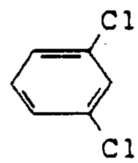 | 135° C |
| 128 | $-CH_2-CH_2-CH_2-$ | H | $-CH_2-CH_2-$ | 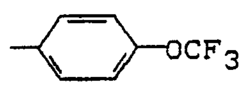 | 132° C |
| 129 | $-CH_2-CH_2-CH_2-$ | H | - | 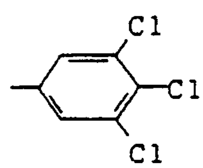 | 196° C |
| 130 | $-CH_2-CH_2-CH_2-$ | H | - | 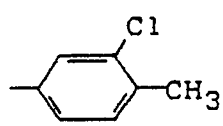 | 176° C |

| Bsp. Nr. | $A^1$ | R | $(A^2)_n$ | Ar | Schmelzpunkt |
|---|---|---|---|---|---|
| 131 | $-(CH_2)_5$ | H | - | 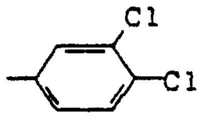 | 131-133° C |
| 132 | $-(CH_2)_5-$ | H | - | 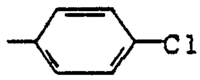 | 149-150° C |
| 133 | $-(CH_2)_5$ | H | $-CH_2-$ | 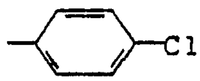 | 130-139° C |
| 134 | $CH_3$<br>\|<br>$-CH-CH_2-$ | H | - | 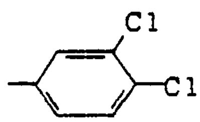 | 146-148° C |
| 135 | $CH_3$<br>\|<br>$-CH-CH_2-$ | H | - | 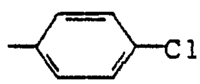 | 160-161° C |
| 136 | $-(CH_2)_4$ | H | - | 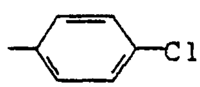 | 156-157° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichsubstanzen eingesetzt:

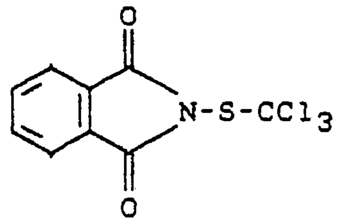

(A)

N-Trichlormethansulfenylphthalsäureimid (Folpet)

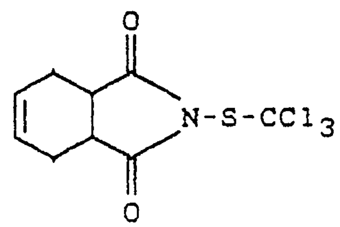

(B)

N-Trichlormethansulfenyltetrahydrophthalsäureimid (Captan)

Beispiel A

Plasmopara-Test (Reben) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Übelregenheit in der Wirksamkeit gegenüber dem Stand der Technik zeigen in diesem Test z.B. die Verbindungen gemäß den Herstellungsbeispielen: 27, 30, 56, 60, 77, 79 und 95.

Beispiel B

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 38, 77, 82 und 87.

## Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Wirksamkeit im Vergleich zu unbehandelten Kontrollpflanzen zeigen in diesem Test z.B. die Verbindungen gemäß den Herstellungsbeispielen: 25, 28, 30, 36, 39, 42, 64, 74, 75, 76, 79 und 82.

## Beispiel D

Bodenbürtige Krankheitserreger-Test (Sojabohne) / Saatgutbehandlung Feldversuch

Saatgutmenge je Parzelle: 30 g
Parzellengröße: 2 $m^2$
Anzahl der Wiederholungen: 3
Entwicklungsstadium der Pflanzen bei der Auswertung: 2-Blatt

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Die Aussaat im Freiland erfolgt nach praxisüblicher Bodenvorbereitung zu einem Zeitpunkt, der den Krankheitsbefall begünstigt.

Die Auswertung erfolgt zu einem Zeitpunkt, bei dem die Krankheitssymptome vollständig und gut ausgeprägt zu erkennen sind.

Eine deutliche Überlegenheit in der Wirksamkeit gegenüber dem Stand der Technik zeigt bei diesem Test z.B. die Verbindung gemäß Herstellungsbeispiel 3.

## Ansprüche

1. N-Substituierte Dichlormaleinimide der allgemeinen Formel (I),

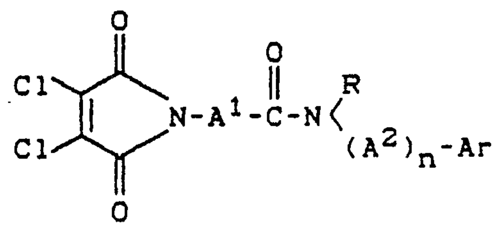

(I)

in welcher
R für Wasserstoff oder Alkyl steht,
Ar für gegebenenfalls substituiertes Aryl steht,
$A^1$ und $A^2$ unabhängig voneinander jeweils für gegebenenfalls substituiertes Alkylen stehen und
n für eine Zahl 0 oder 1 steht,
deren reine Isomere und Isomerengemische, augenommen die Verbindung 2-(Dichlormaleinimido)-acetanilid.

2. N-Substituierte Dichlormaleinimide gemäß Anspruch 1, wobei in der Formel (I)
R für Wasserstoff oder für geradkettiges oder verzweigtes Alkyl mit 1 bis 6 Kohlenstoffatomen steht,
Ar für gegebenenfalls einfach oder mehrefach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Dioxyhalogenalkylen oder Halogenalkylthio mit jeweils 1 bis 4 Kohlenstoffatomen und 1 bis 9 gleichen oder verschiedenen Halogenatomen, jeweils geradkettiges oder verzweigtes Alkenyl, Alkinyl, Alkenyloxy oder Alkinyloxy mit jeweils 3 bis 6 Kohlenstoffatomen, Cycloalkyl mit 3 bis 7 Kohlenstoffatomen, jeweils geradkettiges oder verzweigtes Alkoxycarbonylalkyl oder Alkoxyalkyl mit jeweils 1 bis 4 Kohlenstoffatomen in den einzelnen Alkylteilen oder jeweils gegebenenfalls einfach oder mehrfach, gleich oder verschieden durch Halogen und/oder geradkettiges oder verzweigtes Alkyl mit 1 bis 4 Kohlenstoffatomen substituiertes Phenyl, Benzyl, Phenoxy oder Benzyloxy,
$A^1$ und $A^2$ unabhängig voneinander jeweils für gegebenenfalls durch Phenyl substituiertes, geradkettiges oder verzweigtes Alkylen mit 1 bis 8 Kohlenstoffatomen stehen, wobei der Phenylsubstituent gegebenenfalls einfach oder mehrfach, gleich oder verschieden substituiert sein kann durch: Halogen, jeweils geradkettiges oder verzweigtes Alkyl, Alkoxy, Halogenalkyl oder Halogenalkoxy mit jeweils 1 bis 4 Kohlenstoffatomen und im Fall des Halogenalkyl bzw. Halogenalkoxy mit 1 bis 9 gleichen oder verschiedenen Halogenatomen und
n für eine Zahl 0 oder 1 steht,
deren reine Isomere und Isomerengemische, ausgenommen die Verbindung 2-(Dichlormaleinimido)-acetanilid.

3. N-Substituierte Dichlormaleinimide gemäß Anspruch 1, wobei in der Formel (I)
R für Wasserstoff oder für geradkettiges oder verzweigtes Alkyl mit 1 bis 4 Kohlenstoffatomen steht,
Ar für gegebenenfalls ein- bis fünffach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Trifluormethyl, Trifluormethoxy, Dioxydifluormethylen, Trifluormethylthio, Allyl, Propargyl, Cyclopropyl, Cyclohexyl, Phenyl, Benzyl, Phenoxy oder Benzyloxy,
$A^1$ für gegebenenfalls einfach durch Phenyl substituiertes geradkettiges oder verzweigtes Alkylen mit 1 bis 6 Kohlenstoffatomen steht,
$A^2$ für unsubstituiertes geradkettiges oder verzweigtes Alkylen mit 1 bis 6 Kohlenstoffatomen steht und
n für eine Zahl 0 oder 1 steht,
deren reine Isomere und Isomerengemische, ausgenommen die Verbindung 2-(Dichlormaleinimido)-acetanilid.

4. N-Substituierte Dichlormaleinimide gemäß Anspruch 1, wobei in der Formel (I)
R für Wasserstoff, Methyl oder Ethyl steht,
Ar für gegebenenfalls ein- bis dreifach, gleich oder verschieden substituiertes Phenyl steht, wobei als Substituenten infrage kommen: Fluor, Clor, Brom, Cyano, Nitro, Methyl, Ethyl, n-oder i-Propyl, n-, i-, s- oder t-Butyl, Methoxy, Methylthio, Trifluormethyl, Trifluormethoxy, Trifluormethylthio oder zweifach verknüpftes Dioxydifluormethylen,
A' für gegebenenfalls einfach durch Phenyl substituiertes geradkettiges oder verzweigtes Alkylen mit 1 bis 4 Kohlenstoffatomen steht,
$A^2$ für geradkettiges oder verzweigtes Alkyl mit 1 bis 4 Kohlenstoffatomen steht und
n für eine Zahl 0 oder 1 steht,
deren reine Isomere und Isomerengemische, ausgenommen die Verbindung 2-(Dichlormaleinimido)-acetanilid.

5. Verfahren zur Herstellung von N-substituierten Dichlormaleinimide der allgemeinen Formel (I),

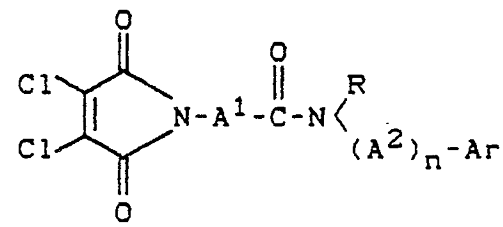 (I)

in welcher
R für Wasserstoff oder Alkyl steht,
Ar für gegebenenfalls substituiertes Aryl steht,
A' und $A^2$ unabhängig voneinander jeweils für gegebenenfalls substituiertes Alkylen stehen und
n für eine Zahl 0 oder 1 steht,
deren reine Isomere und Isomerengemische, ausgenommen die Verbindung 2-(Dichlormaleinimido)-acetanilid, dadurch gekennzeichnet, daß man Dichlormaleinimidcarbonsäurehalogenide der Formel (II),

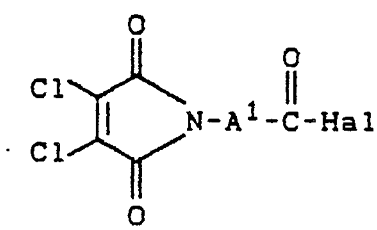 (II)

in welcher
A' die oben angegebene Bedeutung hat und
Hal für Halogen steht,
mit Aminen der Formel (III),

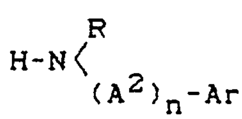 (III)

in welcher
R, Ar, $A^2$ und n die oben angegebene Bedeutung haben,
gegebenenfalls in Gegenwart eines Verdünnungsmittels und gegebenenfalls in Gegenwart eines Säurebindemittels umsetzt.

6. Schädlingsbekämpfungsmittel, gekennzeichnet durch einen Gehalt an mindestens einem N-substituierten Dichlormaleinimid der Formel (I) nach den Ansprüchen 1 und 5.

7. Verwendung von N-substituierten Dichlormaleinimiden der Formel (I) nach den Ansprüchen 1 und 5 zur Bekämpfung von Schädlingen.

8. Verfahren zur Bekämpfung von Schädlingen, dadurch gekennzeichnet, daß man N-substituierte Dichlormaleinimide der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man N-substituierte Dichlormaleinimide der Formel (I) nach den Ansprüchen 1 und 5 mit Streckmitteln und/oder oberflächenaktiven Mitteln vermischt.

Europäisches Patentamt

# EUROPÄISCHER RECHERCHENBERICHT

Nummer der Anmeldung

EP 88 11 8360

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | CH-A- 617 320 (CIBA-GEIGY)<br>* Ansprüche 1,3 *<br>--- | 1,7 | C 07 D 207/456<br>C 07 D 405/12<br>A 01 N 43/36 |
| Y | EP-A-0 117 482 (BAYER)<br>* Ansprüche 1,7 *<br>----- | 1,7 | |
| | | | **RECHERCHIERTE SACHGEBIETE (Int. Cl.4)** |
| | | | C 07 D 207/00<br>C 07 D 405/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 14-02-1989 | CASADO Y MARTIN DE MERCA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)